# FASCICULE DE BREVET EUROPEEN

(11) **EP 3 883 551 B1**
(45) Date de publication et mention de la délivrance du brevet: **13.09.2023**
(21) Numéro de dépôt: 19816550.8
(22) Date de dépôt: 20.11.2019
(51) Int. Cl.: A61K 31/05, A61P 25/28, A23L 33/105, A61K 36/87, A61K 36/45, A61K 45/06, A61K 31/7048, A61K 31/353, A61K 31/192, A23K 10/30, A23K 20/111, A23K 20/163, A23K 20/179, A23K 50/00, A23K 50/20, A23K 50/40, A23L 2/52

(54) **UTILISATION EN PRISE UNIQUE D'UNE COMPOSITION COMPRENANT UN MELANGE PARTICULIER D'EXTRAIT DE RAISINS ET D'EXTRAIT DE BLEUETS**
EINZELDOSISVERWENDUNG EINER ZUSAMMENSETZUNG MIT EINER BESTIMMTEN MISCHUNG AUS TRAUBENEXTRAKT UND HEIDELBEEREXTRAKT
SINGLE-DOSE USE OF A COMPOSITION COMPRISING A PARTICULAR MIXTURE OF GRAPE EXTRACT AND BLUEBERRY EXTRACT

(30) Priorité: 21.11.2018 FR 1871663
(43) Date de publication de la demande: 29.09.2021
(73) Titulaire: Activ'Inside, 33750 Beychac-et-Caillau (FR)
(72) Inventeur: GAUDOUT, David, 33360 CARIGNAN DE BORDEAUX (FR); REY, Stéphane, 26200 MONTELIMAR (FR); LEMAIRE, Benoit, 33500 LIBOURNE (FR); MAZIER, Wilfrid, 33000 BORDEAUX (FR); DUBREUIL, Séverine, 44480 SAUTRON (FR)
(74) Mandataire: Aquinov
(86) Numéro de dépôt international: PCT/EP2019/081945
(87) Numéro de publication internationale: WO 2020/104533

(56) Documents cités:
- WO-A1-2017/072219
- WO-A1-2017/072225
- BENSALEM JULIEN ET AL: "Polyphenols From Grape and Blueberry Improve Episodic Memory in Healthy Elderly with Lower Level of Memory Performance: A Bicentric Double-Blind, Randomized, Placebo-Controlled Clinical Study", THE JOURNALS OF GERONTOLOGY. SERIES A, BIOLOGICAL SCIENCES AND MEDICAL SCIENCESUNITED STATES11 2016,, vol. 74, no. 7, 19 juillet 2018 (2018-07-19), pages 996-1007, XP009514545, ISSN: 1758-535X, DOI: 10.1093/GERONA/GLY166
- Activ Inside Science: "Significant Results on Students Cognitive Performance in only 90 Minutes Organic OPC from Grape Seed Extract", , 26 avril 2019 (2019-04-26), XP055605744, Extrait de l'Internet: URL:https://activinside.com/wp-content/upl oads/2019/05/PRESS-RELEASE.pdf [extrait le 2019-07-16]

## Description

La présente invention concerne l'utilisation en prise unique pour effet rapide et immédiat d'un mélange de molécules spécifiques sur les fonctions cognitives chez l'être humain et l'animal. Un nombre croissant d'adultes sains, et plus particulièrement d'étudiants, recherchent des solutions pour améliorer leurs performances cognitives. Cette quête conduit à une augmentation de l'utilisation inappropriée et parfois dangereuse de stimulants pharmaceutiques. En outre, les solutions nutritionnelles commerciales existantes à utiliser dans le cadre d'une posologie chronique de plusieurs semaines peuvent s'avérer contraignantes et onéreuses.

Il existe donc un besoin relevant d'un intérêt de santé publique, pour une solution sûre, non médicamenteuse, naturelle, et non contraignante pour améliorer les performances cognitives, notamment sous la forme de compléments alimentaires efficaces après une prise unique. Il est également important que la dose soit en adéquation avec une faible quantité d'actifs naturels ainsi qu'une prise unique réalisée peu de temps avant l'effet recherché par exemple avant une épreuve de type examen.

L'objectif de l'invention est de répondre à ces besoins. A cet effet, elle vise l'utilisation d'une composition comprenant un mélange de molécules obtenu à partir de *Vitis vinifera* et de *Vaccinium angustifolium.*

Un mélange de molécules, en particulier riches en flavonoïdes, obtenu à partir de *Vitis vinifera* et de *Vaccinium angustifolium* est déjà connu notamment de la demande FR1560263, ou des demandes WO 2017/072219 et WO2017/072225, qui décrivent l'effet dudit mélange sur les fonctions cognitives de l'être humain lors de prise chronique. De même des apports d'extraits de raisin et de bleuet/myrtille riches en flavonoïdes ont montré une efficacité sur la mémoire lorsqu'ils étaient administrés de façon chronique pendant plusieurs semaines chez des souris, des chiens, et pendant plusieurs mois chez l'Homme.

Or, si les flavonoïdes peuvent s'avérer intéressants pour la cognition en prise chronique, lorsqu'ils sont pris en dose unique, ils n'ont pas forcément d'effet et leur activité est très variable selon le type de flavonoïdes considéré, selon la dose (souvent très importante) et l'âge des personnes concernées. Ainsi deux études cliniques menées avec des boissons cacaotées en administration aigüe (prise unique) ont montré les effets bénéfiques de fortes doses de flavanols de cacao (520, 773, et 994 mg) tandis que le contrôle apportant 46mg de flavanols n'avait aucun effet (Field DT, Williams CM, Butler LT. Consumption of cocoa flavanols results in an acute improvement in visual and cognitive functions. Physiol Behav. 2011 Jun 1;103(3-4):255-60 ; Scholey AB, French SJ, Morris PJ, Kennedy DO, Milne AL, Haskell CF. Consumption of cocoa flavanols results in acute improvements in mood and cognitive performance during sustained mental effort. J Psychopharmacol. 2010 Oct;24(10):1505-14).

Une autre étude menée par Pase et al. (Pase MP, Scholey AB, Pipingas A, Kras M, Nolidin K, Gibbs A, Wesnes K, Stough C. Cocoa polyphenols enhance positive mood states but not cognitive performance: a randomized, placebo-controlled trial. J Psychopharmacol. 2013 May;27(5):451-8) portant également sur la consommation aigüe de flavonoides de cacao ne permet pas de montrer d'amélioration des fonctions cognitives. De même Wightman et al . 2012 ( Wightman EL, Haskell CF, Forster JS, Veasey RC, Kennedy DO. Epigallocatechin gallate, cérébral blood flow parameters, cognitive performance and mood in healthy humans: a double-blind, placebo-controlled, crossover investigation. Hum Psychopharmacol. 2012 Mar;27(2):177-86), ont étudié l'effet d'une consommation aigüe d'extrait de thé vert à des doses de 135mg et 270mg de flavonoides sur la cognition sans qu'aucun résultat significatif ne soit rapporté. Enfin, deux études randomisées, contrôlées (placebo), en double aveugle, Henrickson et Mattes (Hendrickson SJ, Mattes RD. No acute effects of grape juice on appetite, implicit memory and mood. Food Nutr Res. 2008;52) et Haskell Ramsay et al. (Haskell-Ramsay CF, Stuart RC, Okello EJ, Watson AW. Cognitive and mood improvements following acute supplementation with purple grape juice in healthy young adults. Eur J Nutr. 2017 Dec;56(8):2621-2631), ont étudié l'effet aigu d'une consommation de jus de raisin (Vitis Labrusca) sur la cognition (mémoire épisodique, mémoire de travail, attention) chez de jeunes adultes âgés respectivement de 21 ans et 26 ans en moyenne. Dans la première étude 230ml de jus de raisin apportant 138 mg d'anthocyanes et 1504mg de polyphénols ont été consommés tandis que dans la deuxième étude 600ml de jus de raisin contenant 580mg d'anthocyanes et 2 100 mg de polyphénols étaient apportés pour chaque volontaire. Ces deux études ont échoué à montrer un effet sur la mémoire.

Aussi, il est tout à fait surprenant qu'une composition présentant un effet sur la cognition en prise chronique, présente également un effet sur les performances cognitives en prise unique. C'est l'objet de l'invention qui vise en particulier l'utilisation d'une composition comprenant au moins un mélange de molécules obtenu à partir de *Vitis vinifera* et de *Vaccinium angustifolium,* ledit mélange comprenant :
- au moins 1% de catéchines et/ou épicatéchines, le pourcentage étant donné en poids par rapport au poids total du mélange,
- au moins 5 ppm (parties par million dans le mélange) d'acide férulique, et
- au moins 200ppm de resvératrol,
en prise unique chez un être humain sain ou un animal sain pour améliorer ou pour maintenir ses fonctions cognitives.

Avantageusement la prise unique d'une telle composition permet d'obtenir rapidement, en moins de 5 heures, avec une faible dose et un produit naturel, un maintien ou une amélioration des performances cognitives.

D'autres caractéristiques et avantages ressortiront de la description en détails de l'invention qui va suivre, en regard des figures annexées:

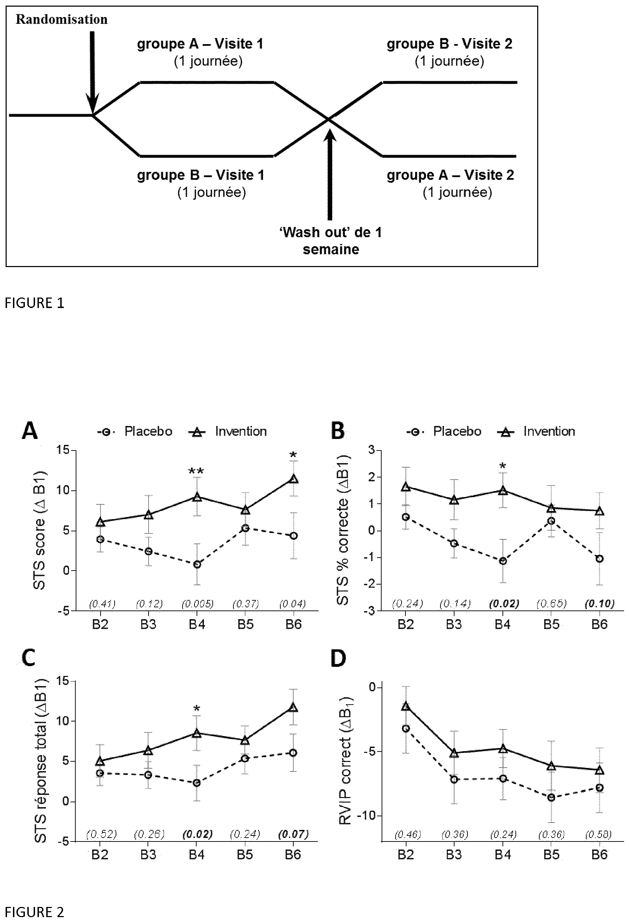

- La Figure 1 représente le plan de l'étude clinique réalisée pour évaluer l'effet selon l'invention.
- La Figure 2 représente des graphes représentant le a variation de la performance par rapport à B1 au cours des répétitions de batterie (B2-B6), regroupée par traitement (P : Placebo, V : Verum). L'évolution du score « net » (coorect-erreur) au test de soustraction en moins trois est représentée en A. Pour ce même test, sont représentés la variation du % de réponses correctes (B) et le nombre de réponse par bloc (C). Les résultats corrects au test de traitement rapide de l'information visuelle sont représentés en D. Les p-valeurs sont celles du paired t-test. Barres verticales représentent l'écart-type.

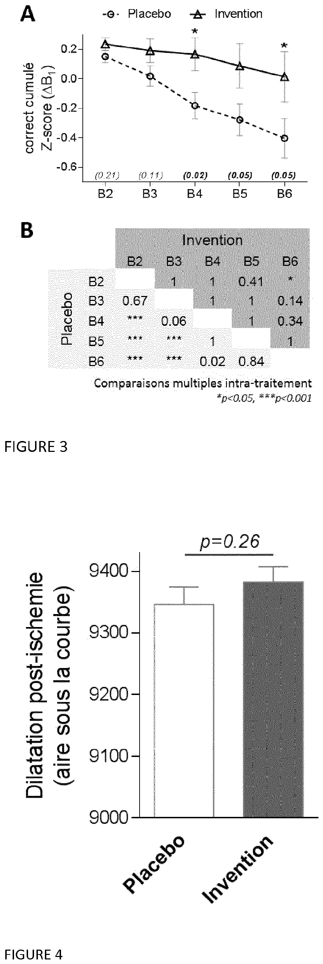

- La Figure 3 représente un graphe représentant le Z-score composite des réponses correctes aux tests STS+SSS+RVIP c'est-à-dire les données représentant la variation du pourcentage du nombre de réponses correctes cumulé par rapport à B1, regroupé par traitement (Placebo, Invention) (A). Les analyses par batterie étaient réalisées avec un paired t-test. Les barres verticales représentent les écart-types. L'évolution au sein des traitements (placebo, Invention) est évaluée par comparaison multiples et les résultats sont retrouvés dans le tableau B.
- La Figure 4 représente les résultats de l'effet du traitement sur le pic de dilatation de l'artère brachiale médié par le flux (60 sec post-ischémie). Paired t-test Placebo vs. Invention, pas de différence.

### DEFINITIONS

Par « animal » au sens de l'invention on entend tout animal pouvant recevoir une composition selon l'invention, par exemple mais de manière non limitative un animal de compagnie, une volaille, un porc, un ruminant, un caprin, ou encore une souris.

Par « anthocyanidine » au sens de l'invention on entend toutes les anthocyanes ou anthocyanines ou anthocyanosides, de forme aglycone ou glycosylée (c'est-à-dire portant des sucres). Ainsi, dans la présente demande et au sens de la présente invention, les termes « anthocyanidine », « anthocyane », « anthocyanines » et « anthocyanosides » sont équivalents.

Par « au moins X% de catéchines et/ou épicatéchines » au sens de l'invention on entend soit au moins X% de catéchines s'il n'y a pas d'épicatéchines dans le mélange, soit au moins X% d'épicatéchines s'il n'y a pas de catéchines dans le mélange, soit au moins X% du mélange de catéchines et d'épicatéchines si à la fois des catéchines et des épicatéchines sont présentes dans le mélange. Préférentiellement, on entend au moins X% du mélange de catéchines et d'épicatéchines.

Par « extrait de *Vaccinium angustifolium »* au sens de l'invention on entend au moins une molécule, préférentiellement un ensemble de molécules, obtenue(s) à partir de *Vaccinium angustifolium.* La matière première peut être les feuilles et/ou les fruits, préférentiellement la matière première est l'ensemble des feuilles et fruits de la plante.

Par « extrait de *Vitis vinifera »* au sens de l'invention on entend au moins une molécule, préférentiellement un ensemble de molécules, obtenue(s) à partir de *Vitis vinifera.* La matière première peut-être les feuilles et/ou les fruits et/ou les pépins et/ou la peau, les bois, préférentiellement la matière première est la partie aérienne de la plante, c'est-à-dire l'ensemble des feuilles, fruits, pellicule (c'est-à-dire la peau), pépins et bois, encore plus préférentiellement la peau (pellicule) et les pépins. L'association de la peau, qui peut être riche en resvératrol, et de pépins, qui peuvent être riches en monomères de flavanols, en oligomères de procyanidines et en proanthocyanidines, peut être particulièrement intéressante pour l'invention.

Par « extrait obtenu à partir d'un mélange de *Vitis vinifera* et de *Vaccinium angustifolium* » au sens de l'invention on entend un ensemble de molécules obtenues à partir d'un mélange de *Vitis vinifera* et de *Vaccinium angustifolium.* La matière première de *Vitis vinifera* peut être les feuilles et/ou les fruits et/ou les pépins et/ les bois, préférentiellement la matière première de *Vitis vinifera* est la partie aérienne de la plante, c'est-à-dire l'ensemble des feuilles, fruits, peau (péllicule), pépins et bois, plus préférentiellement la peau (pellicule) et les pépins. La matière première de *Vaccinium angustifolium* peut être les feuilles et/ou les fruits, préférentiellement la matière première de *Vaccinium angustifolium* est l'ensemble des feuilles et fruits de la plante.

Par « polymère de flavanol » au sens de l'invention on entend un flavanol présentant un degré de polymérisation supérieur à 10.

Par « ppm » au sens de l'invention on entend parties par million (mg/kg) dans le mélange. Sauf indication contraire, ppm se réfère à un poids par rapport au poids total du mélange.

Par « prise unique » au sens de l'invention on entend la consommation d'une ou plusieurs unités de consommation prise en une seule fois.

Par « sain » au sens de l'invention, on entend un être humain ou un animal non atteint d'une pathologie, en particulier d'une pathologie neurologique mais pouvant être fatigué et/ou stressé qui sont des états non pathologiques.

### DESCRIPTION DETAILLEE DE L'INVENTION

L'invention vise donc l'utilisation d'une composition comprenant au moins un mélange de molécules obtenu à partir de *Vitis vinifera* et de *Vaccinium angustifolium,* ledit mélange comprenant :
- au moins 1% de catéchines et/ou épicatéchines, le pourcentage étant donné en poids par rapport au poids total du mélange, préférentiellement au moins 5%, encore plus préférentiellement entre 5% et 50%, notamment entre 7% et 35%,
- au moins 5 ppm (parties par million dans le mélange) d'acide férulique, préférentiellement au moins 10 ppm, encore plus préférentiellement entre 5 ppm et 300 ppm, notamment entre 10 ppm et 100 ppm, et
- au moins 200ppm de resvératrol, préférentiellement au moins 300 ppm, encore plus préférentiellement au moins 400 ppm, encore plus préférentiellement entre 300 ppm et 6000 ppm, notamment entre 400 et 6000 ppm
en prise unique réalisée de façon ponctuelle non répétée chez un être humain ou un animal sain pour améliorer ou pour maintenir ses fonctions cognitives, la prise unique étant réalisée moins de 5 heures avant l'effet recherché.

La composition peut également être utilisée en prise unique pour un effet rapide pour un traitement aigu chez un Homme malade ou un animal malade, en particulier souffrant de maladies pouvant influencer ses performances cognitives telles qu'un rhume par exemple. Ainsi l'invention vise également une composition comprenant au moins un mélange de molécules obtenu à partir de *Vitis vinifera* et de *Vaccinium angustifolium,* ledit mélange comprenant :
- au moins 1% de catéchines et/ou épicatéchines, le pourcentage étant donné en poids par rapport au poids total du mélange, préférentiellement au moins 5%, encore plus préférentiellement entre 5% et 50%, notamment entre 7% et 35%,
- au moins 5 ppm (parties par million dans le mélange) d'acide férulique, préférentiellement au moins 10 ppm, encore plus préférentiellement entre 5 ppm et 300 ppm, notamment entre 10 ppm et 100 ppm, et
- au moins 200ppm de resvératrol, préférentiellement au moins 300 ppm, encore plus préférentiellement au moins 400 ppm, encore plus préférentiellement entre 300 ppm et 6000 ppm, notamment entre 400 et 6000 ppm
pour son utilisation en prise unique réalisée de façon ponctuelle non répétée pour améliorer ou pour maintenir les fonctions cognitives d'un être humain malade ou d'un animal malade.

Cette utilisation est en prise unique est réalisée 5 heures avant l'effet recherché.

Le mélange de molécules de la composition utile selon l'invention comprend préférentiellement également, en plus du au moins 1% de catéchine et/ou épicatéchine, et des au moins 5ppm d'acide férulique, et des au moins 200ppm de resvératrol :
- au moins 50 ppm de quercétine et/ou glycosides de quercétine, préférentiellement au moins 70 ppm de quercétine et/ou glycoside, notamment entre 50 ppm et 10000 ppm, et/ou
- au moins 500 ppm d'anthocyanidines, préférentiellement au moins 600 ppm, encore plus préférentiellement au moins 700 ppm, encore plus préférentiellement entre 600 ppm et 5000 ppm.

Préférentiellement, la malvidine 3 glucoside est l'anthocyanidine prépondérante. De façon préférée, elle est présente à une concentration d'au moins 300ppm dans le mélange.

Selon un premier mode de réalisation, le mélange de molécules est un mélange constitué par un extrait de *Vitis vinifera* et un extrait de *Vaccinium angustifolium.*

Selon un deuxième mode de réalisation, le mélange de molécules est un mélange constitué par un extrait obtenu à partir d'un mélange de *Vitis vinifera* et de *Vaccinium angustifolium.*

Selon un troisième mode de réalisation, le mélange de molécules est un mélange constitué par :
- un extrait de *Vitis vinifera* et/ou un extrait de *Vaccinium angustifolium,* et
- un extrait obtenu à partir d'un mélange de *Vitis vinifera* et de *Vaccinium angustifolium.*

Préférentiellement l'extrait de *Vitis vinifera* présent dans la composition utile selon l'invention est un extrait présentant une teneur en polymères de flavanols inférieure à 0,5% en poids du poids total des polyphénols de l'extrait, encore plus préférentiellement une teneur inférieure à 0,1%. Selon l'invention, les polymères de flavanols sont très peu biodisponibles à l'inverse des monomères de flavanols qui eux sont très rapidement absorbés dans l'intestin grêle puis métabolisés en dérivés méthylés, sulfatés, et glucuronidés. Cette faible présence de polymères est un critère de qualité des extraits de raisin utilisés en particulier pour l'efficacité et la biodisponibilité.

Les extraits selon l'invention peuvent être obtenus par tout procédé permettant d'obtenir un mélange comprenant :
- au moins 1% de catéchine et/ou épicatéchine en poids par rapport au poids total du mélange, préférentiellement au moins 5%, encore plus préférentiellement entre 5% et 50%, notamment entre 7% et 35%,
- au moins 5 ppm d'acide férulique, préférentiellement au moins 10 ppm, encore plus préférentiellement entre 5 ppm et 300 ppm, notamment entre 10 ppm et 100 ppm,
- au moins 200 ppm de resvératrol, préférentiellement au moins 300 ppm, encore plus préférentiellement au moins 400 ppm, encore plus préférentiellement entre 300 ppm et 6000 ppm, notamment entre 400 et 6000 ppm,
- optionnellement, au moins 50 ppm de quercétine et/ou glycosides de quercétine, préférentiellement au moins 70 ppm de quercétine et/ou glycoside, notamment entre 50 ppm et 10000 ppm,
- optionnellement, au moins 500 ppm d'anthocyanidines, préférentiellement au moins 600 ppm, encore plus préférentiellement au moins 700 ppm, encore plus préférentiellement entre 600 ppm et 5000 ppm.

Préférentiellement la malvidine 3 glucoside est l'anthocyanidine prépondérante avec une concentration d'au moins 300ppm. De préférence, les anthocyanidines comprennent au moins 20%, de préférence encore au moins 25% de malvidine 3 glucoside (pourcentage en poids).

Un procédé particulièrement adapté est un procédé comprenant les étapes suivantes :
- obtention d'un extrait de *Vitis vinifera* :
   * extraction à l'eau et/ou à l'éthanol de *Vitis vinifera,* préférentiellement de l'ensemble des feuilles, fruits, pellicule, pépins et bois de *Vitis vinifera;* la quantité de solvant (30% v/v à 96% V/V) mise en oeuvre est comprise entre 2 et 10 fois la masse de matière mise en oeuvre. La durée de l'extraction peut être comprise entre 30 minutes et 24 heures et la température d'extraction comprise entre 20°C et 80°C. Les matières premières utilisées peuvent être sous formes sèches, fraîches, ou congelées entières ou broyées ;
   * séparation de la solution d'eau et/ou éthanol de la matière solide, par exemple par décantation centrifuge ou par pressage et filtration ;
   * évaporation de l'éthanol par évaporation sous-vide à une température préférentiellement inférieure à 60°C et à une pression inférieure à 100mbars ;
   * séparation membranaire de l'extrait préalablement désolvanté de façon à sélectionner préférentiellement les monomères et les oligomères proanthocyanidiques (ayant un degré de polymérisation compris entre 2 et 10 inclus) et d'éliminer les polymères de flavanols (> au décamères), pour obtenir un extrait caractérisé par une teneur en polymères de flavanols inférieure à 0,5% et plus préférentiellement inférieure à 0,1% en poids par rapport au poids total des polyphénols de l'extrait. Cette étape peut être réalisée à l'aide d'une membrane de filtration ayant un seuil de coupure inférieur à 15 000 daltons et plus préférentiellement inférieur à 3000 daltons ;
- obtention d'un extrait de *Vaccinium angustifolium* :
   * extraction à l'eau et/ou à l'éthanol de *Vaccinium angustifolium,* préférentiellement de l'ensemble des feuilles et fruits de *Vaccinium angustifolium;* la quantité de solvant (30% v/v à 96% V/V) mise en oeuvre est comprise entre 2 et 10 fois la masse de matière mise en oeuvre. La durée de l'extraction peut être comprise entre 30minutes et 24heures et la température d'extraction comprise entre 20°C et 80°C. Les matières premières utilisées peuvent être sous formes sèches, fraîches, ou congelées ;
   * séparation de la solution d'eau et/ou éthanol de la matière solide par décantation centrifuge ou par pressage et filtration ;
   * évaporation de l'éthanol par évaporation sous-vide à une température préférentiellement inférieure à 60°C et une pression inférieure à 100mbars ;
   * séchage des extraits par atomisation, étuve sous vide ou lyophilisation avec ou sans support comme une maltodextrine ;
- mélange de l'extrait de *Vitis vinifera* et de *Vaccinium angustifolium* avant ou après l'étape de séchage.

Selon une variante, le procédé consiste en la mise en oeuvre des étapes suivantes :
- mélange de de *Vitis vinifera* et de *Vaccinium angustifolium* extraction à l'eau et/ou à l'éthanol de *Vaccinium angustifolium,* préférentiellement de l'ensemble des feuilles et fruits de *Vaccinium angustifolium,* La quantité de solvant (30% v/v à 96% V/V) mise en oeuvre est comprise entre 2 et 10 fois la masse de matière mise en oeuvre. La durée de l'extraction peut être comprise entre 30minutes et 24heures et la température d'extraction comprise entre 20°C et 80°C. Les matières premières utilisées peuvent être sous formes sèches, fraîches, ou congelées ;
- séparation de la solution d'eau et/ou éthanol du marc solide par décantation centrifuge ou par pressage et filtration ;
- évaporation de l'éthanol par évaporation sous-vide à une température préférentiellement inférieure à 60°C et une pression inférieure à 100mbars ;
- séchage de l'extrait par pulvérisation ou sublimation avec ou sans support comme une maltodextrine.

Quelle que soit la variante du procédé, avant l'étape de séchage, le procédé peut comprendre les étapes suivantes :
- chargement sur une résine des solutions d'extraits mélangées ou non,
- rinçage de la résine avec de l'eau,
- application d'une solution éluante eau/éthanol sur la résine,
- récupération de l'éluât purifié,
- évaporation de l'éthanol dudit éluât,
- concentration dudit éluât
- séchage dudit extrait aqueux purifié.

La composition selon l'invention peut être constituée exclusivement du mélange de molécules, c'est-à-dire des extraits, ou comprendre d'autres constituants. Préférentiellement, en plus du mélange de molécules, la composition selon l'invention contient d'autres constituants, en particulier des excipients ou des agents d'enrobage, tels que de la maltodextrine, de la cellulose microcristalline, des cyclodextrines, de l'amidon, des fibres solubles ou insolubles.

La composition peut se présenter sous toute forme adaptée à une application nutritionnelle, préférentiellement sous forme de poudre.

La composition peut être intégrée dans une autre composition, en particulier dans une composition nutritionnelle se présentant sous une forme choisie parmi les comprimés, les capsules, les gélules, les poudres, les solutions, les microcapsules, les suspensions, les émulsions, les compléments alimentaires, les boissons, et les aliments pour l'Homme ou l'animal.

Il s'agit préférentiellement d'une composition nutritionnelle non thérapeutique destinée à l'Homme comme par exemple des compléments alimentaires, des barres, des produits laitiers, des poudres à avaler ou à réhydrater, des gels, des confitures, des bonbons, des boissons gazeuses ou non, des boissons sèches à réhydrater, des compotes.

Il peut s'agir aussi d'une composition nutritionnelle destinée à l'animal.

Il peut s'agir aussi d'une composition nutritionnelle non thérapeutique destinée à l'animal comme par exemple des aliments secs, tels que des croquettes (extrudées, co-extrudées ou lyophilisées), des friandises (ou treats), des snacks, des aliments humides ou semi-humides tels que des morceaux en sauce, des morceaux en gelée, des boissons, ou encore des compléments alimentaires. De préférence, la composition selon l'invention est intégrée dans des aliments secs tels que des croquettes.

De manière avantageuse, la composition selon l'invention, si elle est destinée à l'animal, peut être intégrée dans une composition, en particulier dans une composition nutritionnelle, en inclusion, c'est-à-dire en l'ajoutant dans la masse de la composition par exemple par imprégnation ou mélange, ou en enrobage, c'est-à-dire en l'appliquant en surface de la composition, par pulvérisation ou par saupoudrage, par exemple en la mélangeant préalablement à un ou plusieurs ingrédients tels que au moins un facteur d'appétence.

Il peut s'agir éventuellement d'une composition pharmaceutique, un médicament ou un produit vétérinaire.

La présence de molécules de *Vitis vinifera* et de molécules de *Vaccinium angustifolium* spécifiques du mélange présentant dans la composition selon l'invention, permet un effet synergique sur le maintien ou l'amélioration des fonctions cognitives.

L'invention a donc pour objet spécifique une composition telle que décrite précédemment, quelle que soit sa variante, pour son utilisation en prise unique chez un être humain ou un animal pour améliorer ou pour maintenir ses fonctions cognitives.

La prise unique est réalisée moins de 5 heures avant l'effet recherché, préférentiellement moins de 3 heures avant l'effet recherché. Cette prise unique peut être réalisée une fois de façon ponctuelle de façon non répétée.

De façon préférée, l'invention vise l'utilisation en prise unique pour améliorer ou pour maintenir ses fonctions cognitives à savoir notamment pour améliorer ou pour maintenir la mémoire et/ou les fonctions exécutives et/ou l'attention et/ou la concentration et/ou l'apprentissage et/ou la flexibilité et /ou la planification et/ou la vigilance.

En particulier, la composition selon l'invention peut être utilisée chez un être humain pour améliorer ou maintenir ses performances à un examen ou pour une épreuve sollicitant les capacités cognitives de façon soutenue.

La composition selon l'invention, en prise unique chez l'être humain, est particulièrement utile et efficace pour maintenir ou améliorer les fonctions cognitives de personnes âgées préférentiellement de plus de 10 ans, encore plus préférentiellement de plus de 15 ans et encore plus préférentiellement de plus de 18 ans. Il peut s'agir notamment d'étudiants et d'adultes actifs.

La composition selon l'invention, en prise unique chez l'animal, est particulièrement utile et efficace pour maintenir ou améliorer les fonctions cognitives des chiens, des chats, ou des chevaux.

Préférentiellement, la composition selon l'invention est utilisée à une quantité permettant de fournir à l'Homme ou l'animal :
- au moins 100 µg par kg de poids corporel de catéchine et/ou épicatéchine, de façon préférentielle 500 µg/kg et de façon encore plus préférentielle 1mg/kg, et
- préférentiellement au moins 0,05 µg par kg de poids corporel d'acide férulique, de façon préférentielle 0.25 µg/kg et de façon encore plus préférentielle 0.5µg/kg, et
- au moins 10 µg par kg de poids corporel de resvératrol, de façon préférentielle 50 µg/kg et de façon encore plus préférentielle 100µg/kg, et
- éventuellement au moins 0,2 µg par kg de poids corporel de quercétine et/ou de glycosides quercétine, de façon préférentielle 1 µg/kg et de façon encore plus préférentielle 2µg/kg, et
- éventuellement au moins 1 µg par kg de poids corporel d'anthocyanidines, et de façon préférentielle 5 µg/kg et de façon encore plus préférentielle 10µg/kg.

L'invention est à présent illustrée à travers des exemples et résultats d'essais démontrant l'effet synergique en prise unique et rapide sur les fonctions cognitives de la composition objet de la présente demande.

### EXEMPLES

### Exemple 1 : Composition selon l'invention

Ce premier exemple de mélange selon l'invention est obtenu par la mise en oeuvre du procédé tel que décrit en suivant.

Les matières premières utilisées sont :
- la pellicule et les graines (pépins) de fruits de *Vitis vinifera,* sous-produits de l'industrie vinicole,
- des baies congelées de *Vaccinium angustifolium.*

400 g de baies congelées de *Vaccinium angustifolium* sont broyées et mélangées avec une solution de 2000ml d'éthanol 80% (V/V) avec une teneur de 0,1% en poids de HCl. Le mélange est maintenu à température ambiante (20°c) pendant 24 heures. La solution éthanolique est ensuite séparée de la pulpe grâce à une filtration, et concentrée sous vide avec un évaporateur rotatif à 20% de matière sèche. Une partie de cet extrait est conservée pour être testée, l'autre partie est conservée pour être mélangée à l'extrait de *Vitis vinifera.*

500 g de pellicule et de pépins de *Vitis vinifera* sont mélangés avec 2500 ml d'éthanol 80% (V/V) avec une teneur de 0,1% en poids de HCl à 40°C pendant 5 heures. La solution éthanolique est ensuite séparée de la pulpe grâce à une filtration. L'éthanol est ensuite éliminé sous vide avec un évaporateur rotatif à une température de 50°C sous 60mbars. La solution aqueuse est ensuite diluée pour avoir une matière sèche de 5% et filtrée sur une membrane de 5000 daltons. Le perméat obtenu est alors chargé sur une colonne de résine (C18) à 1BV/heure. La résine est ensuite rincée une première fois avec 3BV d'eau distillée à 2BV/heure, et ensuite éluée avec 5BV d'une solution éthanolique à 80% (V/V) à 1 BV/heure. Une partie de la solution extraite est conservée pour être testée et caractérisée (Tableau 1a). Les polyphénols présentés dans ce tableau ont été mesurés par chromatographie liquide ultra haute performance avec un détecteur fluorescence.

**Tableau la : Contenu en flavanols de l'extrait de Vitis vinifera**

| Teneur en monomères de flavanols et en proanthocyanidines (% de poids sec - eq. Epicatechines) (mesuré par LC avec un détecteur fluorescence) | |
|---|---|
| Monomères | 35,27% ± 1,67 |
| Dimeres | 22,92% ± 1,31 |
| Trimères | 8,93% ± 0,46 |
| Tétramères | 2,95% ±0,14 |
| Pentamères | 1,09% ± 0,07 |
| Hexamères | 0,63% ±0,14 |
| Heptamères | 0,15%± 0,05 |
| Octamères | Non Détécté |
| Nonamères | Non Détécté |
| Décamères | Non Détécté |
| Polymères (Degré de polymérisation >10) | Non Détécté |

L'autre partie est ensuite mélangée avec l'extrait de *Vaccinium angustifolium* pour former le mélange selon l'invention et une maltodextrine est ajoutée au mélange jusqu'à obtenir une solution présentant un taux de matière sèche de 30%.

La solution est ensuite séchée par pulvérisation avec une température d'entrée de 160°C.

Le produit obtenu est une poudre mauve contenant les polyphénols présentés dans le tableau 1b. Les polyphénols présentés dans ce tableau ont été mesurés par chromatographie liquide ultra haute performance masse/masse (UPLC-MS/MS).

**Tableau 1b : Contenu en polyphénols de la compositions selon l'invention de l'exemple 1**

| | Mélange selon l'invention | 500mg de mélange/kg de poids corporel souris | Equivalent en mg/kg (Homme selon la FDA) |
|---|---|---|---|
| Catéchine et épicatéchine | 25,7% | 128,5 mg/kg de poids corporel | 10,4 mg/kg de poids corporel |
| Anthocyanidines (dont malvidine 3 glucoside) | 0,436% (1310ppm) | 2,18 mg/kg de poids corporel | 177µg/kg de poids corporel |
| Quercetine et glycosydes de quercétine | 0,864% | 4,32 mg/kg de poids corporel | 35µg/kg de poids corporel |
| Acide férulique | 0,0094% | 47µg /kg de poids corporel | 3,82µg/kg de poids corporel |
| Resvératrol | 0,0437% | 218 µg/kg de poids corporel | 17,7µg/kg de poids corporel |

### Exemple 2 : Composition selon l'invention

Ce deuxième exemple de mélange selon l'invention est obtenu par la mise en oeuvre du procédé tel que décrit en suivant.

Les matières premières utilisées sont :
- les pépins et la peau de *Vitis vinifera,*
- des baies de Vaccinium angustifolium.

1000g de marc congelé de *Vaccinium angustifolium* sont broyés et mélangés avec une solution de 5000ml d'éthanol 60% (V/V) avec une teneur de 0,1% en poids de HCl. Le mélange est maintenu à température ambiante (20°c) pendant 24 heures. La solution éthanolique est ensuite séparée de la pulpe grâce à une filtration, et concentrée sous vide avec un évaporateur rotatif à 20% de matière sèche.

400 g de peau et de pépins de *Vitis vinifera* sélectionnés sont mélangés avec 1500 ml d'éthanol 80% (V/V) à 60°C pendant 5 heures. La solution éthanolique est ensuite séparée de la pulpe grâce à une filtration. L'éthanol est ensuite éliminé sous vide avec un évaporateur rotatif à une température de 50°C sous 60mbars. La solution aqueuse est ensuite diluée pour avoir une matière sèche de 5% et filtrée sur une membrane de 5000 daltons. Le perméat obtenu est alors chargé sur une colonne de résine (C18) à 1BV/heure. La résine est ensuite rincée une première fois avec 3BV d'eau distillée à 2BV/heure, et ensuite éluée avec 5BV d'une solution éthanolique à 80% (V/V) à 1 BV/heure.

Une partie de la solution extraite est conservée pour être testée et caractérisé (tableau 2a).

**Tableau 2a: Contenu en flavanols de l'extrait de Vitis vinifera**

| Teneur en monomères de flavanols et en proanthocyanidines (% de poids sec - eq. Epicatechines) (mesuré par LC avec un détecteur fluorescence) | |
|---|---|
| Monomères | 9,4% ± 0,8 |
| Dimeres | 4,0% ± 0,3 |
| Trimères | 0,81% ± 0,1 |
| Tétramères | 0,24% ± 0,1 |
| Pentamères | Non Détécté |
| Hexamères | Non Détécté |
| Heptamères | Non Détécté |
| Octamères | Non Détécté |
| Nonamères | Non Détécté |
| Décamères | Non Détécté |
| Polymères (Degré de polymérisation >10) | Non Détécté |

Le produit obtenu est une poudre mauve contenant les polyphénols présentés dans le tableau 2b. Les polyphénols présentés dans ce tableau ont été mesurés par chromatographie liquide ultra haute performance (UPLC-MS/MS).

**Tableau 2b : Contenu en polyphénols de la composition selon l'invention de l'exemple 2**

| | Mélange selon l'invention | 4mg de mélange/kg poids corporel souris | Equivalent en mg/kg (Homme selon la FDA) |
|---|---|---|---|
| Catéchine et épicatéchine | 9,4% | 376 µg/ kg de poids corporel | 124 µg/ kg de poids corporel |
| Anthocyanidines (dont malvidine 3 glucoside) | 700ppm (328 ppm) | 2.8 µg/ kg de poids corporel | 1.6µg/ kg de poids corporel |
| Quercetine et glycosydes de quercétine | 77ppm | 0.37µg/ kg de poids corporel | 0.21µg/ kg de poids corporel |
| Acide férulique | 20ppm | 0.08 µg/ kg de poids corporel | 0.044µg/ kg de poids corporel |
| Resvératrol | 5327ppm | 21 µg/ kg de poids corporel | 12µg/ kg de poids corporel |

### Exemple 3 : Composition selon l'invention avec excipients destinée à l'être humain

19,980 kg de la composition de l'exemple 1 est mélangé avec 0,020kg de Silice colloidale. La composition est obtenue par mélange des constituants dans les conditions classiques connues de l'homme du métier. La composition est conditionnée dans un sac PET lui-même conditionné dans un carton.

**Tableau 3 : Contenu en polyphénols de la composition selon l'invention de l'exemple 3**

| | Mélange selon l'invention | 500mg de mélange/kg de poids corporel souris | Equivalent en mg/kg (Homme selon la FDA) |
|---|---|---|---|
| Catéchine et épicatéchine | 25,7% | 128,5 mg/kg de poids corporel | 10,4 mg/kg de poids corporel |
| Anthocyanidines (dont malvidine 3 glucoside) | 0,436% (1310ppm) | 2,18 mg/kg de poids corporel | 177µg/kg de poids corporel |
| Quercetine et glycosydes de quercétine | 0,864% | 4,32 mg/kg de poids corporel | 35µg/kg de poids corporel |
| Acide férulique | 0,0094% | 47µg /kg de poids corporel | 3,82µg/kg de poids corporel |
| Resvératrol | 0,0437% | 218 µg/kg de poids corporel | 17,7µg/kg de poids corporel |

### Exemple 4: Composition nutritionnelle selon l'invention destinée à l'être humain

Les matières premières utilisées sont :
- raisins frais de *Vitis vinifera,* utilisés pour la fabrication du vin,
- des baies congelées de *Vaccinium angustifolium.*

7,2 g de baies congelées de *Vaccinium angustifolium* sont broyés puis pressés. Le jus est séparé de la fraction solide (1,8g). La fraction solide est mélangée avec une solution de 35ml d'éthanol 80% (V/V) avec une teneur de 0,1% en poids de HCl. Le mélange est maintenu à température ambiante (20°c) pendant 24 heures. La solution éthanolique est ensuite séparée de la pulpe grâce à une filtration, et concentrée sous vide avec un évaporateur rotatif à 20% de matière sèche. L'extrait est alors séché sous vide sur un support maltodextrine. 180mg d'extrait sous forme de poudre sont alors récoltés pour être mélangés à l'extrait de *Vitis vinifera.*

300g de raisins frais sont pressés, le jus étant séparé de la matière solide (71g) constituée de pellicule et de pépins de *Vitis vinifera.* La matière solide est mélangée avec 1000 ml d'éthanol 80% (V/V) avec une teneur de 0,1% en poids de HCl à 40°C pendant 5 heures. La solution éthanolique est ensuite séparée de la pulpe grâce à une filtration. L'éthanol est ensuite éliminé sous vide avec un évaporateur rotatif à une température de 50°C sous 60mbars. La solution aqueuse est ensuite diluée pour avoir une matière sèche de 5% et filtrée sur une membrane de 5000 daltons. Le perméat obtenu est alors chargé sur une colonne de résine (C18) à 1BV/heure. La résine est ensuite rincée une première fois avec 3BV d'eau distillée à 2BV/heure, et ensuite éluée avec 5BV d'une solution éthanolique à 80% (V/V) à 1 BV/heure. La solution hydroalcoolique est alors évaporée avec un évaporateur rotatif à une température de 50°C sous 60mbars et l'extrait obtenu est alors séché sous vide. 420mg d'extrait de *Vitis vinifera* sec sont alors récupérés puis mélangés aux 180 mg d'extrait de *Vaccinium angustifolium* pour constituer 600mg le produit selon l'invention.

Le produit selon l'invention est administré sous la forme de gélules. Chaque gélule contient un total de 450mg de poudre, composés de 300mg de produit selon l'invention additionné avec 150mg de maltodextrine de maïs.

La quantité quotidienne de produit préconisée est de 600mg, soit 2 gélules.

**Tableau 4 : Contenu en polyphénols de la composition selon l'invention de l'exemple 4**

| | Mélange selon l'invention | Dose quotidienne de produit/kg (Homme) (600mg/62kg de poids moyen) |
|---|---|---|
| Catéchine et épicatéchine | 20.57% | 1.99mg/ kg de poids corporel |
| Anthocyanidines | 0.11% | 10.6µg/ kg de poids corporel |
| Quercetine et glycosydes de quercétine | 0.18% | 17.4µg/ kg de poids corporel |
| Acide férulique | >5ppm | >0.5µg/ kg de poids corporel |
| Resvératrol | 1100ppm | 10.6µg/ kg de poids corporel |

### Exemple 5: Composition nutritionnelle selon l'invention destinée à l'être humain

L'exemple 5 est une gélule de 400mg, constituée par
- Composition de l'exemple 1 : 300m
- Vitamine C : 80mg
- Maltodextrine : 20mg

La composition est obtenue par mélange des constituants dans les conditions classiques connues de l'homme du métier, et mis dans une gélule selon les conditions classiques également.

La quantité préconisée est de 1 à 2 gélules par jour.

### Exemple 6 : Composition nutritionnelle selon l'invention destinée à l'animal

La composition selon l'invention de l'exemple 2 a été ajoutée à une croquette sèche extrudée pour chien respectant les normes de l'AFCO et comprenant des farines animales, de la matière grasse, des fibres, des céréales et des agents conservateurs et antioxydants.

L'ajout de la composition à la croquette a été fait selon plusieurs modes de réalisation, notamment par enrobage et par inclusion.

Des essais en enrobage ont été effectués en ajoutant la composition selon l'invention à un facteur d'appétence liquide D'Tech volaille (SPF, Elven, France). Une première couche de matière grasse de volaille (6% par rapport au poids de la croquette) a été ajoutée en enrobage sur la croquette, suivie par une couche de mélange entre le facteur d'appétence (1%, 2% ou 3%, le % étant par rapport au poids de la croquette) et la composition selon l'invention (0,02%, 0,04% ou 0,1%, le % étant par rapport au poids de la croquette).

Des essais en inclusion ont été effectués en ajoutant la composition selon l'invention (0,02%, 0,04% ou 0,1%, le % étant par rapport au poids de la croquette) dans la matière première (aussi appelé prémix) avant extrusion.

### EVALUATION DE L'EFFET EN PRISE UNIQUE DE LA COMPOSITION SELON INVENTION SUR LES FONCTIONS COGNITIVES ET LA FONCTION ENDOTHELIALE

Une étude clinique monocentrique, en cross over, double aveugle et contre placebo, a été menée sur 30 étudiants volontaires sains âgés de 18 à 25 ans.

La composition du produit à l'étude, est celle de l'exemple 4.

Les objectifs de cette étude portaient sur l'effet en prise unique du produit selon l'invention, d'une part sur les fonctions cognitives (mémoire de travail et attention) mesurées par des tests réalisés à l'aide d'une batterie informatique validée (COMPASS), et d'autre part sur la fonction endothéliale évaluée par mesure de la dilatation médiée par le flux (FMD).

En effet, on sait que le principal mécanisme d'action connue de l'efficacité sur les fonctions cognitives d'une composition est lié à une amélioration de la fonction endothéliale, et plus particulièrement une augmentation de la disponibilité en monoxyde d'azote (NO). Soit par augmentation de sa synthèse par les cellules endothéliales, soit par une limitation de sa dégradation (enzymes, espèces oxydatives réactives). Le NO joue un rôle majeur dans l'augmentation du flux sanguin cérébral et par conséquent sur la vasodilatation locale au cours de l'activation neuronale. Une méthode simple et non invasive pour évaluer la fonction endothéliale consiste à mesurer la dilatation du diamètre de l'artère brachiale médiée par le flux (FMD) en réponse à une ischémie transitoire.

La composition de l'exemple 4 a été administrée en une prise unique orale de 2 gélules (300mg/gélule soit 600mg/prise). Le placebo était une gélule de même aspect contenant uniquement de la maltodextrine (dépourvue de polyphénol). Le plan de l'étude est présenté dans la Figure 1.

Les sujets devaient respecter les consignes suivantes:
- Pendant toute la durée de l'étude : ne pas modifier leurs habitudes, en particulier en matière d'alimentation, sommeil, activités physiques et intellectuelles ; ne pas consommer de complément alimentaire; ne pas fumer ni consommer de stupéfiant; compléter un carnet de suivi du temps de sommeil tous les jours.
- Pendant les 24 h précédant chaque visite d'évaluation (V1 et V2): respecter les restrictions alimentaires permettant de modérer la consommation de polyphenols dans les 24heures précédant les tests, compléter un journal de suivi alimentaire.
- Ne pas pratiquer plus de 2h d'activité physique intense la veille d'une visite d'évaluation.
- Se rendre à jeun depuis 12 h aux visites d'évaluation.
- Rapporter l'enquête alimentaire et le journal de suivi du sommeil aux visites d'évaluation.

### Evaluation de la cognition

Après contrôle des critères d'inclusion et d'exclusion, les sujets intégraient l'étude et étaient soumis à une session d'entraînement sur la batterie d'évaluation cognitive informatique mise en place pour cette étude à partir de module du logiciel COMPASS (Université Nothumbria, Newcasttle). L'objectif de cette batterie était de modéliser les conditions d'un examen scolaire/universitaire. Pour se faire, une série de tests informatisés longue et exigeante sur le plan cognitif a été utilisée. Celle-ci consistait en une suite de six batteries comportant chacune : 2 min de soustraction par trois (STS), 2 min de soustraction par sept (SSS), 5min de traitement rapide d'informations visuelles (RVIP) et 2 min d'évaluation subjective par échelle visuelle analogique (performance, anxiété, performance, fatigue). Ci-dessous est listé le détail des objectifs et déroulement des tâches :
Soustraction de 3 (STS):
   Cette tâche permettait de tester l'attention et la mémoire de travail du sujet. Cela consiste à demander au participant de réaliser des soustractions en -3 le plus rapidement possible et le plus précisément possible en écrivant la réponse à l'aide d'un clavier informatique. Un nombre initialement tiré au hasard entre 800 et 999 est affiché à l'écran. Nombre qui disparait dès la première entrée tapée par le participant. Dans le cas où une réponse erronée est donnée, la réponse suivante correcte sera automatiquement déterminée sur la base de la dernière réponse donnée par le participant. La tâche dure 2 minutes.
Soustraction de 7 (SSS) :
   Cette tâche permettait également d'évaluer l'attention et la mémoire de travail du sujet. Le fonctionnement de cette tâche est identique à STS à la différence près qu'il est demandé au participant de réaliser des soustractions en 7. La tâche dure également 2 minutes.
Tâche de traitement rapide d'informations visuelles (RVIP):
   Cette tâche visait à mesurer les capacités d'attention des participants. Tout au long du test, des chiffres apparaissaient un à un à l'écran (100/min). Il était demandé de détecter l'occurrence de 3 chiffres pairs ou impairs consécutifs et d'appuyer sur la barre d'espace de leur clavier aussi vite que possible quand une suite était détectée. La tâche a une durée de 5 minutes.

La durée totale de cette batterie à haute exigence cognitive était de 66 minutes : durant ces 66 minutes les sujets enchaînent six répétitions d'une batterie comportant STS, SSS, RVIP puis une évaluation subjective. Chaque batterie dure 11 minutes.

### Evaluation de la fonction endothéliale

### Procédure de mesure de la FMD

### Préparation des sujets :

Les participants de l'étude étaient à jeun depuis 12h et avaient déclarés avoir respecté les consignes. Pour chaque participant, les mesures des FMD ont été réalisées à la même heure lors des 2 visites.

### Équipement :

Les enregistrements ont été réalisés par le même opérateur dans une salle dédiée, , avec un échographe (VINNO TM E10) et une sonde linéaire (VINNO TM F4-42L) à 10 MHz.

### Acquisition de l'image :

Le sujet était accompagné jusqu'à la salle d'examen FMD par un technicien de recherche clinique, puis installé sur le lit d'examen médical, et laissé au repos allongé confortablement sur le dos pendant au moins 15 minutes. Après la prise de la tension artérielle, le bras droit était immobilisé. Le brassard à tension était placé au niveau de l'avant-bras, au-dessus du poignet. La sonde d'enregistrement était placée et fixée par le système de maintien sur le bras du sujet juste au-dessus du pli du coude afin d'avoir une image nette de l'artère brachiale (diamètre maximal) sans modification de l'orientation de la sonde.
Dilatation médiée par le flux: FMD

L'image longitudinale de l'artère a été enregistrée pendant 2 minutes (divisées en 4 séquences de 30 secondes consécutives en raison de la limitation de la durée possible d'enregistrement par l'échographe). Puis pour provoquer la phase d'ischémie temporaire, l'opérateur a gonflé le brassard de tension à une pression supra systolique de 230 mmHg pendant 6 minutes. Immédiatement après relâchement de la compression du brassard, l'image de l'artère a été à nouveau enregistrée pendant 2 minutes (divisées en 4 séquences de 30 secondes consécutives).

A la fin du 1^{er} examen échographique de la 1^{ère} visite (V1), une mesure de la distance entre la position de la sonde et le pli du coude à l'aide d'une règle centimétrique a permis de réaliser pour un même sujet des enregistrements localisés au même endroit de l'artère brachiale lors des mesures suivantes pour une meilleure reproductibilité.

### Procédure d'analyse des enregistrements

L'analyse des enregistrements a été effectuée avec le logiciel « Vascular Research Tools », module « Brachial Analyzer », équipé du système de détection semi-automatique des parois vasculaires. Toutes les analyses ont été effectuées par le même opérateur qui était en aveugle des produits pris.

La FMD a été calculée comme étant le pourcentage d'augmentation du diamètre de l'artère par rapport à sa valeur de base, selon la formule suivante: FMD= (diamètre maximal-diamètre basal)/diamètre basal ×100. La valeur du diamètre basal de l'artère a été calculée comme étant la moyenne des valeurs obtenues au cours des 2 minutes avant la compression. La valeur du diamètre maximal a été calculée comme étant la moyenne la plus haute des 4 valeurs (moyenne 30sec) mesurées après relâchement de la compression.

### Analyses statistiques

### Analyses des données des tests cognitifs

L'homogénéité des scores et d'éventuelles intéractions *traitement*/*ordre* ont été déterminées en réalisant des ANOVA à mesures répétées (rANOVA) sur chaque test à la B₁.

Pour chaque type de test, l'évolution de la performance au cours des six répétitions de la batterie était estimé en réalisant des rANOVA sur la variation des scores (variation brute du nombre de réponse correctes par rapport à B1).

La méthode du score composite ou « Zⁱ-*composite*» a été utilisée, afin d'obtenir une mesure unique prenant en compte l'ensemble des scores corrects obtenus au différents tests évaluant l'attention et la mémoire de travail : STS, SSS, RVIP. Le Zⁱ-*composite* a permis d'avoir pour chaque individu et à chaque batterie (bloc) une quantification de la performance cognitive globale. Pour obtenir le Zⁱ-*composite* d'un sujet « *i* », les résultats de chaque test étaient centrés et réduits (Z-score) afin d'obtenir leur distance par rapport à la moyenne du groupe en fonction de l'écart-type, notés Zⁱ_{STS}, Zⁱ_{SSS}, et Zⁱ_{RVIP}. Cette étape de normalisation permet ensuite de calculer la moyenne des tests à regrouper, appelée ici Zⁱ*composite* [(Zⁱ_{STS} + Zⁱ_{SSS} + Zⁱ_{RVIP})/3)].

### Analyses des données de FMD

La reproductibilité échographique a été évaluée en calculant la variabilité individuelle intra-visite et inter-visite. Les variables quantitatives ont été décrites en calculant les moyennes et les écart-types. L'effet du traitement était évalué en réalisant des test t de Student (appariés et non-appariés selon conditions).

L'ensemble des tests statistiques portant sur les tests cognitifs ont été réalisés en utilisant R (support Jupyter.org). Les tests sur les données de FMD étaient effectués via le logiciel Prism6 (GraphPad). Tous les tests statistiques réalisés dans cette étude étaient bilatéraux avec un interval de confiance fixé à 5%.

### Résultats

### Caractéristiques de la population

Les caractéristiques initiales des 26 sujets de la population ITT sont présentées dans le tableau 5 ci-dessous :

**Tableau 5 : Caractéristiques de la population ITT avec mesures cognitives validées**

| Variables | Valeurs (n=30) |
|---|---|
| Age | 22.0 ±1.7 années |
| Sexe F :H | 14 :16 |
| IMC | 21.3±2.2 kg/m2 |
| PAS (V0) | 115.2 ±9.6 mmHg |
| PAD (V0) | 74.6±7.6 mmHg |
| FC (V0) | 77.6±14.8 bpm |
| Durée moyenne de sommeil | 7.9±0.8 heures |

| | |
|---|---|
| F: Femme ; H: Homme; IMC: Indice de Masse Corporelle; POP: pilule oeso-progestative; PAS: Pression artérielle systolique ; PAD: Pression artérielle diastolique; FC: Fréquence cardiaque; V0: Visite d'inclusion. | |

Au sein de cette population ITT, les valeurs des analyses FMD de 4 sujets n'étaient pas interprétables à tous les points. Ces 4 sujets ont donc été sortis de la population ITT pour les statistiques FMD, amenant ainsi le nombre de sujets à 26 ayant des caractéristiques similaires à la population ITT totale (Tableau 6).

**Tableau 6 : Caractéristiques de la population ITT avec mesures FMD validées**

| Variables | Valeurs (n=26) |
|---|---|
| Age | 22,2 ±1,75 années |
| Sexe F :H | 14 :12 |
| IMC | 21,3±2,42 kg/m2 |
| Traitement | POP n=13, Nexplanon n=1 |
| PAS (V0) | 115,3 ±9,62 mmHg |
| PAD (V0) | 74,7±7,11 mmHg |
| FC (V0) | 76,3±14,57 bpm |
| Durée moyenne de sommeil | 8±0,85 heures |

| | |
|---|---|
| F: Femme ; H: Homme; IMC: Indice de Masse Corporelle; POP: pilule oeso-progestative; PAS: Pression artérielle systolique; PAD: Pression artérielle diastolique; FC: Fréquence cardiaque; V0: Visite d'inclusion. | |

### Evolution des scores (STS,SSS, RVIP) au cours du temps

L'évolution de la performance cognitive au fil de la batterie de tests était suivie via la variation du score net par bloc (correct-erreur) par rapport à B₁ (Figure 2 A). Au fil des répétitions des tâches de STS, le score STS des sujets était augmenté avec l'invention, significativement à partir de B₄, alors que ce n'était pas le cas sous placebo. L'analyse plus fine des résultats a montré que cette amélioration de la performance nette était en fait liée au maintien du nombre de réponses correctes tout au long du test (Figure 2 B) couplé à une augmentation du nombre de proposition sous la composition selon l'invention (Figure 2 C). Malgré un aspect visuel pouvant sembler favorable au traitement avec l'invention, aucun résultat significatif n'a été mis en évidence sur les SSS et RVIP (Figure 2 D).

### Evolution de la « Performance globale » au cours du temps

Afin d'obtenir un score global des scores corrects aux tests d'attention et de mémoire de travail effectués (STS, SSS, RVIP), la méthode du Zⁱ-*composite* a été employée. La normalisation puis combinaison des différents résultats a montré une différence significative de performance globale (Figure 3A). En effet, de B₄ à B₆, les scores sous Verum étaient significativement plus élevés que placebo. De plus, en comparant l'évolution au sein des traitements (Bonferroni bloc *vs* bloc), on remarque que les scores sous placebo diminuaient de façon significative alors que sous Verum le niveau de performance globale était maintenu de la B₁ à la B₆ (Figure 3 B).

### Résultats sur la fonction endothéliale

### Qualité des enregistrements FMD

*Variabilité individuelle intra-visite* : calculée en prenant pour un même sujet le premier diamètre basal comparé au deuxième diamètre basal de la visite. Les valeurs individuelles sont ensuite moyennées pour obtenir une variation individuelle moyenne intra-visite observée à 1.21 ±5.2 % (52 valeurs).

*Variabilité individuelle inter-visite* : calculée en prenant pour un même sujet, le premier diamètre basal de la V1 comparé à la même mesure en V2. Les valeurs individuelles sont ensuite moyennées pour obtenir une variation moyenne inter-visite observée à 0,51 ±4.14% (26 valeurs).

### Effet aigu de l'invention sur la FMD

Aucune différence statistiquement significative entre les traitements sur le pic de FMD (à 60 sec post-ischiémie) n'a été observée avec une moyenne à 5,93% sous l'invention et 5,73% sous placebo (p=0,45). L'amplitude globale de la dilation post-ischémie n'était pas significativement différente selon le traitement (Figure 4).

### Conclusion de l'étude

Cette étude menée chez des étudiants (jeunes adultes) sains, a permis d'évaluer l'impact d'une supplémentation en prise unique d'une composition selon l'invention sur le maintien de la performance cognitive au cours d'une batterie de tests longue et exigeante sur le plan cognitif. De manière surprenante, la combinaison et la quantité spécifique de polyphénols présents dans la composition selon l'invention permet d'obtenir un effet aigu sur la cognition. Les résultats de cette étude montrent que les résultats du test de mémoire de travail sont améliorés avec l'invention via une amélioration « qualitative » (plus de réponses correctes) et « quantitative » (plus de tentatives). En comparaison, les mêmes sujets sous placebo présentaient une diminution des performances de mémoire de travail et d'attention au fil des répétitions de la batterie de tests. L'administration en prise unique d'une composition selon l'invention permet donc une amélioration de la performance globale au cours d'un challenge cognitif prolongé (simulation examen). De façon intéressante, les effets sur les fonctions cognitives ont atteint leur maximum lors de la batterie B4, soit environ 2 heures après la prise du produit.

Or, dans ces mêmes conditions, aucun changement significatif concomitant de la fonction endothéliale n'a été observé. Ainsi l'effet n'est pas lié à une amélioration de la perfusion des structures cérébrales engagées dans l'exécution des tâches de ces tests.

Par conséquent, il apparaît que les résultats de la présente étude en ce qui concerne les effets en prise unique et effet rapide de la composition sur les fonctions cognitives, n'étaient pas prévisibles et que l'homme de l'art aurait pu s'attendre à ce qu'un effet du produit sur la FMD soit également observé, par rapport au placebo.

### En effet :

- La dose de flavonoïdes testée ( ≥ 174 mg) était inférieure à la plus petite dose (520 mg de flavonoïdes de cacao) pour laquelle un effet aigu sur des fonctions cognitives a été mis en évidence dans l'art antérieur
- La composition selon l'invention n'a pas induit d'effet significatif sur la FMD, par rapport au placebo;
- Les précédents travaux menés sur ce produit ne permettent pas d'appuyer l'hypothèse d'un effet antioxydant significatif après une prise unique ;
- Les autres mécanismes d'action potentiels de ce produit (action sur la neurogénèse et la plasticité neuronale) mettent en jeu des processus longs, qui ne sont pas susceptibles de se traduire par des effets physiologiques significatifs après seulement quelques heures ;

Plusieurs études cliniques menées sur des produits issus du bleuet ou du raisin n'avaient mis en évidence aucun effet de ces produits sur les fonctions cognitives, après une prise unique.

## Revendications

1. Utilisation d'une composition comprenant un mélange de molécules obtenu à partir de *Vitis vinifera* et de *Vaccinum angustifolium,* ledit mélange comprenant :
- au moins 1% de catéchines et/ou épicatéchines, le pourcentage étant donné en poids par rapport au poids total du mélange,
- - au moins 5 ppm (parties par million dans le mélange) d'acide férulique, et
- - au moins 200ppm de resvératrol,
en prise unique réalisée de façon ponctuelle non répétée chez un être humain sain ou un animal sain pour améliorer ou pour maintenir ses fonctions cognitives, la prise unique étant réalisée moins de 5 heures avant l'effet recherché.

2. Utilisation selon la revendication 1, **caractérisée en ce que** la prise unique est réalisée moins de 3 heures avant l'effet recherché.

3. Utilisation selon l'une des précédentes revendications, pour améliorer ou pour maintenir la mémoire et/ou les fonctions exécutives et/ou l'attention et/ou la concentration et/ou l'apprentissage et/ou la flexibilité et /ou la planification et/ou la vigilance et/ou pour améliorer ou maintenir ses performances à un examen ou pour une épreuve sollicitant les capacités cognitives de façon soutenue.

4. Utilisation selon l'une des précédentes revendications, **caractérisée en ce que** le mélange de molécules utilisé est un mélange de molécules comprenant :
- au moins 5% de catéchines et/ou épicatéchines, le pourcentage étant donné en poids par rapport au poids total du mélange, et/ou
- au moins 10 ppm (parties par million dans le mélange) d'acide férulique.

5. Utilisation selon l'une des précédentes revendications, **caractérisée en ce que** le mélange de molécules utilisé est un mélange de molécules constitué par :
- un extrait de *Vitis vinifera* et/ou un extrait de *Vaccinium angustifolium,* et
- un extrait obtenu à partir de *Vitis vinifera* et de *Vaccinium angustifolium.*

6. Utilisation selon la précédente revendication, **caractérisée en ce que** l'extrait de *Vitis vinifera* présent dans le mélange de molécules utilisé présente une teneur en polymères de flavanols inférieure à 0,5% en poids total des polyphénols de l'extrait.

7. Utilisation selon l'une des précédentes revendications, **caractérisée en ce que** le mélange de molécules utilisé est un mélange de molécules comprenant également :
- la malvidine 3 glucoside à une concentration d'au moins 300ppm, et/ou
- au moins 50ppm de quercétine et/ou glycosides de quercétine, et/ou
- au moins 500ppm d'anthocyanidines.

8. Utilisation selon l'une des précédentes revendications, **caractérisée en ce que** la composition comprenant le mélange de molécules est prise en une prise unique permettant de fournir à l'être humain ou l'animal :
- au moins 100 µg par kg de poids corporel de catéchines et/ou d'épicatéchines et/ou
- au moins 0,05 µg par kg de poids corporel d'acide férulique et/ou
- au moins 1 µg par kg de poids corporel de resvératrol.

9. Utilisation selon l'une des précédentes revendications, **caractérisée en ce que** la composition comprenant le mélange de molécules est prise en une prise unique permettant de fournir à l'être humain ou l'animal :
- au moins 500 µg par kg de poids corporel de catéchines et/ou d'épicatéchines et/ou
- au moins 0,25 µg par kg de poids corporel d'acide férulique et/ou
- au moins 5 µg par kg de poids corporel de resvératrol.

10. Utilisation selon l'une des précédentes revendications, **caractérisée en ce que** la composition comprenant le mélange de molécules est prise en une prise unique permettant de fournir à l'être humain ou l'animal :
- au moins 1 mg par kg de poids corporel de catéchines et/ou d'épicatéchines et/ou
- au moins 0,5 µg par kg de poids corporel d'acide férulique et/ou
- au moins 10 µg par kg de poids corporel de resvératrol.

11. Utilisation selon la revendication 7, **caractérisée en ce que** la composition comprenant le mélange de molécules est prise en une prise unique permettant de fournir à l'être humain ou l'animal :
- au moins 0,2 µg par kg de poids corporel de quercétine et/ou glycosides quercétines,
- au moins 1 µg par kg de poids corporel d'anthocyanidines.

12. Utilisation selon l'une des précédentes revendications, **caractérisée en ce que** la composition comprenant le mélange de molécules se présente sous une forme choisie parmi les comprimés, les capsules, les gélules, les poudres, les solutions, les microcapsules, les suspensions, les émulsions, les compléments alimentaires, les boissons, et les aliments pour l'être humain ou l'animal.

13. Utilisation selon l'une des précédentes revendications, chez des êtres humains sains âgées de plus de 10 ans.

14. Utilisation selon l'une des revendications 1 à 12, chez des animaux sains choisis parmi les chiens, les chats et les chevaux.

15. Composition comprenant un mélange de molécules obtenu à partir de *Vitis vinifera* et de *Vaccinum angustifolium,* ledit mélange comprenant :
- au moins 1% de catéchines et/ou épicatéchines, le pourcentage étant donné en poids par rapport au poids total du mélange,
- au moins 5 ppm (parties par million dans le mélange) d'acide férulique, et
- au moins 200 ppm de resvératrol,
pour une utilisation thérapeutique en prise unique réalisée de façon ponctuelle non répétée pour améliorer ou pour maintenir les fonctions cognitives d'un être humain malade ou d'un animal malade, la prise unique étant réalisée moins de 5 heures avant l'effet recherché.

## Patentansprüche

1. Verwendung einer Zusammensetzung, umfassend eine Mischung aus Molekülen, die ausgehend von *Vitis vinifera* und von *Vaccinum angustifolium* erhalten wurde, wobei die Mischung umfasst:
- mindestens 1 % Catechine und/oder Epicatechine, wobei der Prozentsatz als Gewicht bezogen auf das Gesamtgewicht der Mischung gegeben ist,
- - mindestens 5 ppm (Teile pro Million in der Mischung) Ferulasäure, und
- - mindestens 200 ppm Resveratrol,
als Einzeldosis, die auf nicht wiederholte punktuelle Weise bei einem gesunden Menschen oder einem gesunden Tier erfolgt, um seine kognitiven Funktionen zu verbessern oder beizubehalten, wobei die Einzeldosis weniger als 5 Stunden vor der beabsichtigten Wirkung erfolgt.

2. Verwendung nach Anspruch 1, **dadurch gekennzeichnet, dass** die Einzeldosis weniger als 3 Stunden vor der beabsichtigten Wirkung erfolgt.

3. Verwendung nach einem der vorstehenden Ansprüche, um das Gedächtnis und/oder die exekutiven Funktionen und/oder die Aufmerksamkeit und/oder die Konzentration und/oder das Lernen und/oder die Flexibilität und/oder die Planung und/oder die Wachsamkeit zu verbessern oder beizubehalten und/oder seine/ihre Leistungen bei einem Examen oder bei einer Prüfung, welche die kognitiven Fähigkeiten auf anhaltende Weise fordert, zu verbessern oder beizubehalten.

4. Verwendung nach einem der vorstehenden Ansprüche,
**dadurch gekennzeichnet, dass** die Mischung von verwendeten Molekülen eine Mischung von Molekülen ist, umfassend:
- mindestens 5 % Catechine und/oder Epicatechine, wobei der Prozentsatz als Gewicht bezogen auf das Gesamtgewicht der Mischung gegeben ist, und/oder
- mindestens 10 ppm (Teile pro Million in der Mischung) Ferulasäure.

5. Verwendung nach einem der vorstehenden Ansprüche,
**dadurch gekennzeichnet, dass** die Mischung von verwendeten Molekülen eine Mischung von Molekülen ist, die aus Folgendem besteht:
- einem Extrakt aus *Vitis vinifera* und/oder einem Extrakt aus *Vaccinium angustifolium,* und
- einem Extrakt, der ausgehend von *Vitis vinifera* und von *Vaccinium angustifolium* erhalten wird.

6. Verwendung nach dem vorstehenden Anspruch,
**dadurch gekennzeichnet, dass** der Extrakt aus *Vitis vinifera,* der in der Mischung von verwendeten Molekülen vorhanden ist, einen Gehalt an Flavanolpolymeren von weniger als 0,5 % des Gesamtgewichts von Polyphenolen des Extrakts aufweist.

7. Verwendung nach einem der vorstehenden Ansprüche,
**dadurch gekennzeichnet, dass** die Mischung von verwendeten Molekülen eine Mischung von Molekülen ist, die auch Folgendes umfassen:
- Malvidin-3-glucosid in einer Konzentration von mindestens 300 ppm, und/oder
- mindestens 50 ppm Quercetin und/oder Glycoside von Quercetin, und/oder
- mindestens 500 ppm Anthocyanidine.

8. Verwendung nach einem der vorstehenden Ansprüche,
**dadurch gekennzeichnet, dass** die Zusammensetzung, welche die Mischung von Molekülen umfasst, als eine Einzeldosis eingenommen wird, was es ermöglicht, dem Menschen oder dem Tier Folgendes bereitzustellen:
- mindestens 100 µg pro kg Körpergewicht Catechine und/oder Epicatechine und/oder
- mindestens 0,05 µg pro kg Körpergewicht Ferulasäure und/oder
- mindestens 1 µg pro kg Körpergewicht Resveratrol.

9. Verwendung nach einem der vorstehenden Ansprüche,
**dadurch gekennzeichnet, dass** die Zusammensetzung, welche die Mischung von Molekülen umfasst, als eine Einzeldosis eingenommen wird, was es ermöglicht, dem Menschen oder dem Tier Folgendes bereitzustellen:
- mindestens 500 µg pro kg Körpergewicht Catechine und/oder Epicatechine und/oder
- mindestens 0,25 µg pro kg Körpergewicht Ferulasäure und/oder
- mindestens 5 µg pro kg Körpergewicht Resveratrol.

10. Verwendung nach einem der vorstehenden Ansprüche,
**dadurch gekennzeichnet, dass** die Zusammensetzung, welche die Mischung von Molekülen umfasst, als eine Einzeldosis eingenommen wird, was es ermöglicht, dem Menschen oder dem Tier Folgendes bereitzustellen:
- mindestens 1 mg pro kg Körpergewicht Catechine und/oder Epicatechine und/oder
- mindestens 0,5 µg pro kg Körpergewicht Ferulasäure und/oder
- mindestens 10 µg pro kg Körpergewicht Resveratrol.

11. Verwendung nach Anspruch 7, **dadurch gekennzeichnet, dass** die Zusammensetzung, welche die Mischung von Molekülen umfasst, als eine Einzeldosis eingenommen wird, was es ermöglicht, dem Menschen oder dem Tier Folgendes bereitzustellen:
- mindestens 0,2 µg pro kg Körpergewicht Quercetin und/oder Quercetinglycoside,
- mindestens 1 µg pro kg Körpergewicht Anthocyanidine.

12. Verwendung nach einem der vorstehenden Ansprüche,
**dadurch gekennzeichnet, dass** die Zusammensetzung, welche die Mischung von Molekülen umfasst, in einer Form vorliegt, die ausgewählt ist aus Tabletten, Kapseln, Gelatinekapseln, Pulvern, Lösungen, Mikrokapseln, Suspensionen, Emulsionen, Nahrungsergänzungsmitteln, Getränken und Lebensmitteln für den Menschen oder das Tier.

13. Verwendung nach einem der vorstehenden Ansprüche bei gesunden Menschen, die älter als 10 Jahre sind.

14. Verwendung nach einem der Ansprüche 1 bis 12 bei gesunden Tieren, ausgewählt aus Hunden, Katzen und Pferden.

15. Zusammensetzung, umfassend eine Mischung von Molekülen, die ausgehend von *Vitis vinifera* und *Vaccinum angustifolium* erhalten wurde, wobei die Mischung umfasst:
- mindestens 1 % Catechine und/oder Epicatechine, wobei der Prozentsatz als Gewicht bezogen auf das Gesamtgewicht der Mischung gegeben ist,
- mindestens 5 ppm (Teile pro Million in der Mischung) Ferulasäure und
- mindestens 200 ppm Resveratrol, für eine therapeutische Verwendung als Einzeldosis auf nicht wiederholte punktuelle Weise, um die kognitiven Funktionen eines kranken Menschen oder eines kranken Tiers zu verbessern oder beizubehalten, wobei die Einzeldosis weniger als 5 Stunden vor der beabsichtigten Wirkung erfolgt.

## Claims

1. Use of a composition comprising a mixture of molecules obtained from *Vitis vinifera* and *Vaccinum angstifolium,* said mixture comprising:
- at least 1% catechins and/or epicatechins, the percentage being given by weight relative to the total weight of the mixture,
- - at least 5 ppm (parts per million in the mixture) ferulic acid, and
- - at least 200 ppm resveratrol,
in a single dose taken on a one-off, non-repeated basis by a healthy human being or a healthy animal in order to improve or maintain cognitive functions, the single dose being taken less than 5 hours before the desired effect.

2. Use according to claim 1, **characterized in that** the single dose is taken less than 3 hours before the desired effect.

3. Use according to either of the preceding claims for improving or maintaining memory and/or executive functions and/or attention and/or concentration and/or learning and/or flexibility and/or planning and/or vigilance and/or for improving or maintaining performance during an examination or for a test involving sustained cognitive abilities.

4. Use according to any of the preceding claims, **characterized in that** the mixture of molecules used is a mixture of molecules comprising:
- at least 5% catechins and/or epicatechins, the percentage being given by weight relative to the total weight of the mixture, and/or
- at least 10 ppm (parts per million in the mixture) ferulic acid.

5. Use according to any of the preceding claims, **characterized in that** the mixture of molecules used is a mixture of molecules consisting of:
- a *Vitis vinifera* extract and/or a *Vaccinium angstifolium* extract, and
- an extract obtained from *Vitis vinifera* and *Vaccinium angstifolium.*

6. Use according to the preceding claim, **characterized in that** the *Vitis vinifera* extract present in the mixture of molecules used has a flavanol polymer content of less than 0.5% by total weight of the polyphenols of the extract.

7. Use according to any of the preceding claims, **characterized in that** the mixture of molecules used is a mixture of molecules also comprising:
- malvidin 3 glucoside at a concentration of at least 300 ppm, and/or
- at least 50 ppm quercetin and/or quercetin glycosides, and/or
- at least 500 ppm anthocyanidins.

8. Use according to any of the preceding claims, **characterized in that** the composition comprising the mixture of molecules is taken in a single dose which makes it possible for human beings or animals to be provided with:
- at least 100 µg per kg of body weight catechin and/or epicatechin, and/or
- at least 0.05 µg per kg of body weight ferulic acid, and/or
- at least 1 µg per kg of body weight resveratrol.

9. Use according to any of the preceding claims, **characterized in that** the composition comprising the mixture of molecules is taken in a single dose which makes it possible for human beings or animals to be provided with:
- at least 500 µg per kg of body weight catechin and/or epicatechin, and/or
- at least 0.25 µg per kg of body weight ferulic acid, and/or
- at least 5 µg per kg of body weight resveratrol.

10. Use according to any of the preceding claims, **characterized in that** the composition comprising the mixture of molecules is taken in a single dose which makes it possible for human beings or animals to be provided with:
- at least 1 mg per kg of body weight catechin and/or epicatechin, and/or
- at least 0.5 µg per kg of body weight ferulic acid, and/or
- at least 10 µg per kg of body weight resveratrol.

11. Use according to claim 7, **characterized in that** the composition comprising the mixture of molecules is taken in a single dose which makes it possible for human beings or animals to be provided with:
- at least 0.2 µg per kg of body weight quercetin and/or quercetin glycosides,
- at least 1 µg per kg of body weight anthocyanidins.

12. Use according to any of the preceding claims, **characterized in that** the composition comprising the mixture of molecules is in a form selected from tablets, capsules, gel capsules, powders, solutions, microcapsules, suspensions, emulsions, food supplements, beverages, and foods for human beings or animals.

13. Use according to any of the preceding claims in healthy human beings of more than 10 years old.

14. Use according to any of claims 1 to 12 in healthy animals selected from dogs, cats and horses.

15. Composition comprising a mixture of molecules obtained from *Vitis vinifera* and *Vaccinum angstifolium,* said mixture comprising:
- at least 1% catechins and/or epicatechins, the percentage being given by weight relative to the total weight of the mixture,
- at least 5 ppm (parts per million in the mixture) ferulic acid, and
- at least 200 ppm resveratrol, for therapeutic use in a single dose taken on a one-off, non-repeated basis in order to improve or maintain cognitive functions of a sick human being or a diseased animal, the single dose being taken less than 5 hours before the desired effect.
